(19) **Europäisches Patentamt / European Patent Office / Office européen des brevets**

(11) **EP 1 777 284 B1**

(12) **FASCICULE DE BREVET EUROPEEN**

(45) Date de publication et mention de la délivrance du brevet:
**01.10.2008 Bulletin 2008/40**

(51) Int Cl.:
*C10G 69/04* (2006.01)
*C10G 69/14* (2006.01)
*C07C 11/06* (2006.01)
*C10G 11/05* (2006.01)

(21) Numéro de dépôt: **06291545.9**

(22) Date de dépôt: **02.10.2006**

(54) **Procédé de conversion directe d'une charge comprenant des oléfines à quatre, et/ou cinq atomes de carbone, pour la production de propylène avec une co-production d'essence désulfurée à haut indice d'octane**

Verfahren zur Direktumwandlung eines Olefine mit mindestens vier oder fünf Kohlenstoffatomen enthaltenden Einsatzstoffs zur Herstellung von Propylen und von entschwefeltem Benzin mit hoher Oktanzahl

Process for the direct conversion of a cut containing C4 and/or C5 for the production of propylene and co-production of a high-octane number desulfurised gasoline

(84) Etats contractants désignés:
**BE DE FR IT NL**

(30) Priorité: **19.10.2005 FR 0510727**

(43) Date de publication de la demande:
**25.04.2007 Bulletin 2007/17**

(73) Titulaire: **Institut Français du Pétrole**
**92852 Rueil Malmaison Cédex (FR)**

(72) Inventeurs:
• **Coupard, Vincent**
  **69120 Vaulx en Velin (FR)**
• **Surla, Karine**
  **69560 Saint Cyr sur le Rhône (FR)**

(56) Documents cités:
**FR-A- 2 875 234**     **FR-A- 2 879 620**
**US-A- 4 392 002**     **US-A- 6 049 017**
**US-A1- 2005 222 475**

EP 1 777 284 B1

**Description**

**Domaine de l'invention**

L'invention concerne un procédé permettant de produire du propylène et une essence désulfurée à haut indice d'octane à partir d'une charge d'hydrocarbures comprenant des oléfines dont le nombre de carbone est majoritairement égal à 4 ou 5, cette charge d'hydrocarbures provenant d'une part de la coupe C4/C5 issue d'une unité de vapocraquage, et d'autre part de la coupe essence d'une unité de craquage catalytique (notée FCC).

Les proportions de chacune de ces deux coupes peuvent être quelconque.

Le terme vapocraquage désigne l'unité de craquage à la vapeur d'eau de coupes hydrocarbonées diverses, le plus souvent une coupe dite naphta de point d'ébullition compris entre 100°C et 350°C, et produisant majoritairement des oléfines, essentiellement de l'éthylène et du propylène, mais aussi des oléfines à nombre d'atome de carbone plus élevé.

La coupe oléfinique C4/C5 issue d'une unité de vapocraquage est généralement produite avec un rendement pouvant aller selon les charges et les conditions opératoires jusqu'à 10% poids, et n'est pas immédiatement valorisable.

Il est donc tout à fait intéressant de pouvoir la convertir en propylène et en essence dans un procédé optimisé à cette fin, qui est l'objet de la présente invention.

Le terme FCC désigne le procédé de craquage catalytique en lit fluidisé de fractions pétrolières de point d'ébullition supérieur à environ 350 °C, généralement un distillat sous vide, éventuellement de l'huile désasphaltée ou un résidu atmosphérique.

L'essence de FCC correspond à une coupe de point d'ébullition généralement compris entre 70°C et 250°C. Cette essence est relativement riche en composés insaturés de type oléfinique, et contient du soufre jusqu'à des valeurs pouvant atteindre quelques % poids.

L'essence de FCC nécessite un traitement spécifique de désulfuration avant d'être incorporée au pool essence, traitement faisant appel à une unité d'hydrogénation sélective, notée SHU, et une unité d'hydrodésulfuration notée HDT.

La présente invention concerne donc un procédé de production de propylène et de coproduction d'une essence désulfurée et à fort indice d'octane, le dit procédé combinant une unité d'oligocraquage fonctionnant en une étape, et un ensemble de désulfuration comprenant une unité d'hydrogénation sélective (SHU) et une unité d'hydrodésulfuration (HDT).

On entend dans la suite du texte par unité d'oligocraquage en une étape un procédé dans lequel s'effectue à la fois l'oligomérisation d'oléfines légères, typiquement de C4 à C6 formant des oléfines lourdes, typiquement de C8 à C12, et le craquage simultané de ces oléfines lourdes en oléfines majoritairement plus légères que celles de la charge de départ, principalement du propylène.

Le catalyseur utilisé pour réaliser l'oligocraquage en une étape est est un catalyseur dédié qui permet d'être plus sélectif en propylène sans avoir de craquage des paraffines éventuellement contenues dans la charge. De plus c'est un procédé plus économique que les procédés en deux étapes qui séparent l'étape d'oligomérisation des oléfines légères, et l'étape de craquage des oléfines lourdes précédemment formées.

Dans la suite du texte et conformément au vocabulaire de l'homme du métier, les termes coupes C4, C5, Cn, désignent des ensembles de molécules hydrocarbonées possédant majoritairement 4, 5 ou n atomes de carbone. Bien entendu, la délimitation de ces ensembles appelés coupes n'est pas stricte, et dans une coupe C5, par exemple, se trouve un certain pourcentage de coupes adjacentes C4 et C6. En fonction de la qualité des unités de séparation, le plus souvent des distillations, ce pourcentage peut être limité à moins de 5% poids.

Une coupe large caractérisée par un intervalle, par exemple C4/C6, signifie l'ensemble des molécules hydrocarbonées comprises entre C4 et C6 inclus, avec la même remarque que précédemment sur la teneur en coupes adjacentes, en l'occurrence C3 et C7.

**Examen de l'art antérieur**

La présente invention décrit un procédé de production de propylène à partir d'une coupe oléfinique majoritairement en C4/C5, et d'une essence de FCC, qui peuvent être introduites dans des proportions quelconques.

- Le brevet français FR 2 608 595 décrit le procédé de métathèse qui convertit en propylène un mélange éthylène + n-butène.

Un des avantages du procédé selon l'invention par rapport à celui de la métathèse, est de produire du propylène à partir des composés oléfiniques contenus dans les coupes $C_4$/C5, et de ne pas requérir de consommation massive d'éthylène, produit de coût élevé. En outre, s'il est appliqué sur un site de vapocraquage, le procédé selon l'invention permet non seulement de ne pas utiliser d'éthylène comme charge, mais de coproduire de l'éthylène avec le propylène.

Ceci permet d'augmenter le ratio global propylène sur éthylène, ce qui est conforme aux tendances du marché.

- Le procédé décrit dans la demande internationale WO 01/04237 est un autre procédé de production de propylène en une étape à partir d'oléfines légères utilisant un catalyseur comprenant une zéolithe ZSM-5 à l'état fluidisé.

Les conditions opératoires typiques de ce procédé sont une température au voisinage de 600°C, et une pression de 0,1 MPa à 0,2 MPa ( 1 MPa = 10 bars).

Dans ces conditions, le rendement en propylène est d'environ 30 %, et peut augmenter jusqu'à 50 % avec le recyclage des coupes C4 et C5 qui n'ont pas réagi.

Un inconvénient de ce procédé est que la technologie en lit fluidisé est onéreuse du point de vue des investissements, et nécessite une conduite du procédé relativement délicate. Elle conduit également à des pertes notables de catalyseur par attrition.

- Dans la famille des procédés en une étape (c'est à dire sans oligomérisation préalable des fractions C4/C5), on peut également citer un procédé dont on trouvera une description dans l'article «Production of Propylène from Low Valued Olefins» (qu'on peut traduire par "Production de propylène à partir d'oléfines de faible valeur"), paru dans la revue "Hydrocarbon Engineering" (Ingénierie des hydrocarbures) de mai 1999.

Il s'agit d'un procédé en lit fixe dont le catalyseur est une zéolithe de type ZSM-5 agissant en présence de vapeur d'eau.

La température est voisine de 500°C, et la pression est comprise entre 0,1 MPa et 0,2 MPa. La durée de cycle annoncée est de l'ordre de 1000 heures. Le catalyseur est régénéré in situ, et sa durée de vie globale, c'est à dire la période pendant laquelle il est utilisé dans le réacteur avant son renouvellement complet, est d'environ 15 mois.

Le rendement en propylène annoncé est de 40 % environ, et pourrait monter à 60 % avec le recyclage des coupes C4 et C5 n'ayant pas réagi.

Ce procédé permet d'obtenir un rendement en propylène relativement élevé.

Il requiert cependant l'utilisation de quantités élevées de vapeur d'eau.

- On peut également citer un procédé décrit dans la demande internationale WO 99/29805, et dans les brevets ou demandes de brevet EP-0921181 et EP-A-0921179.

Il s'agit d'un procédé utilisant un catalyseur zéolithique de type MFI ayant un rapport Si/Al élevé (de 180 à 1000) pour limiter les réactions de transfert d'hydrogène responsables de la production de diènes et d'aromatiques.

La température est voisine de 550°C, la pression voisine de 0,1MPa, et la vitesse spatiale comprise entre 10 h-1 et 30 h-1. Ce procédé peut être mis en oeuvre dans des réacteurs en lit fixe, mobile ou fluidisé.

Le catalyseur utilisé comporte une zéolithe de type MFI dont le rapport Si/Al (rapport atomique Silicium/Aluminium) est supérieur ou égal à 180, et de préférence une zéolithe ZSM-5 de rapport Si/Al compris entre 300 et 1000.

- On peut enfin citer le procédé décrit dans la demande de brevet EPA 1 195 424 A1.

Il s'agit d'un procédé utilisant un catalyseur zéolithique de type MFI ayant un rapport Si/Al de 180 à 1000, ou un catalyseur zéolithique de type MEL ayant un rapport Si/Al de 150 à 800, ces rapports Si/Al élevés étant nécessaires pour limiter les réactions de transfert d'hydrogène responsables de la production de diènes et d'aromatiques.

La température est comprise entre 500°C et 600°C, la pression partielle d'oléfines est comprise entre 0,01 MPa et 0,2 MPa, et la vitesse spatiale est comprise entre 5 h-1 et 30 h-1.

- Le brevet US 6,049,017 décrit un procédé de production de propylène à partir d'une coupe oléfinique comportant la succession d'étapes suivante: a) une séparation des mono oléfines et des dioléfines de la coupe de départ, b) une séparation des normales et des iso oléfines réalisée sur le flux de mono oléfines faisant appel à un agent oxydant et à un catalyseur acide,

c) un craquage des normales oléfines faisant appel à un catalyseur à petits pores.

US 2005/0222475 décrit également un procédé de production de propylène et de coproduction d'essence.

Le procédé décrit dans la présente invention ne fait appel à aucune unité de séparation des normales et des iso oléfines, et de plus, conduit non seulement à la formation de propylène, mais aussi à la formation de quantité d'essence additionnelle d'excellente qualité appelé dans le contexte de la présente invention coproduction d'essence désulfurée à haut indice d'octane

Par essence à haut indice d'octane, on entend un indice d'octane MON supérieur à 80 et un indice d'octane RON supérieur à 92.

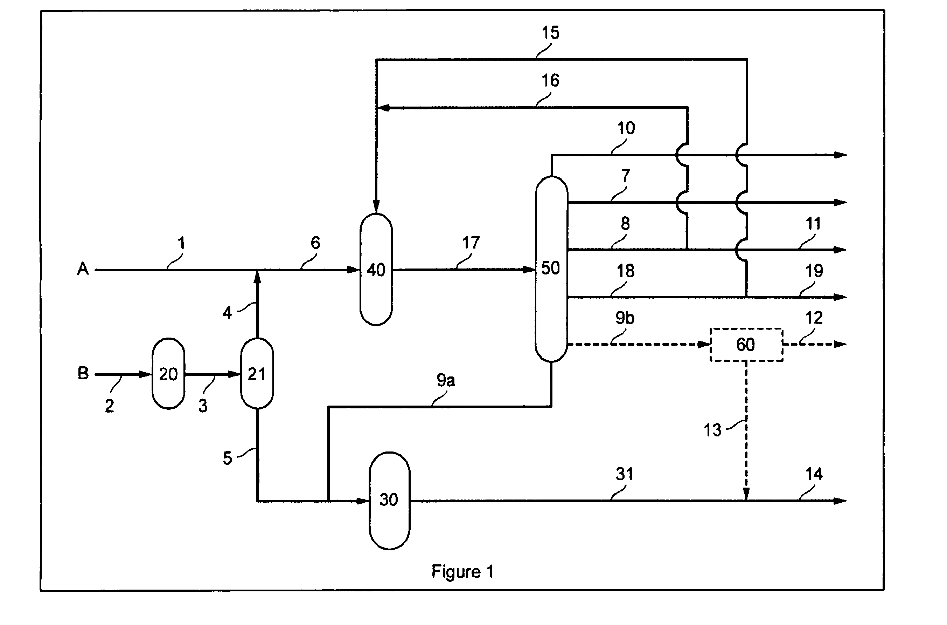

### Description sommaire de la figure1

La figure 1 représente le schéma de base du procédé selon l'invention et permet une compréhension plus aisée de la description détaillée qui va suivre.

Sur la figure 1, les lignes en traits pleins représentent les unités ou flux obligatoires, et les lignes en traits pointillés représentent les unités ou flux facultatifs.

### Description sommaire de l'invention

La présente invention concerne un procédé de production de propylène et de coproduction d'une essence désulfurée à haut indice d'octane à partir d'une charge constituée d'une part d'une coupe C4/C5 issue d'une unité de vapocraquage (A), et d'autre part d'une essence de FCC (B), le dit procédé comportant les étapes suivantes:

- une étape d'hydrogénation sélective (20) de l'essence de FCC (B) correspondant au flux (2), produisant un effluent (3) envoyé dans une unité de séparation (21) qui produit deux fractions, une fraction légère (4) qui est envoyée dans une unité d'oligocraquage en une étape (40), et une fraction lourde majoritairement en C6+ (5), qui est envoyée dans une unité d'hydrotraitement (30),
- une étape d'oligocraquage en une étape (40) qui traite en mélange la coupe C4/C5 issue de l'unité de vapocraquage (A), correspondant au flux (1), et la fraction légère (4) issue de l'unité de séparation (21), l'effluent (17) de l'unité d'oligocraquage (40) étant séparé dans une ou plusieurs colonnes à distiller (50) en au moins 5 coupes:

> 1) une coupe de tête (10) constituée majoritairement d'éthylène
> 2) une coupe de tête (7) qui constitue la production en propylène du procédé,
> 3) une coupe intermédiaire (8) constituée majoritairement de molécules en C4, dont une partie (16) est recyclée à l'entrée de l'unité d'oligocraquage en une étape (40), et dont la partie non recyclée (11) est valorisée en gaz de pétrole liquéfié,
> 4) une coupe intermédiaire (18) constituée majoritairement de molécules en C5 et C6, dont une partie (15) est recyclée à l'entrée de l'unité d'oligocraquage en une étape (40), et dont la partie non recyclée (19) constitue une essence dirigée vers le pool essence,
> 5) une coupe de fond (9a) constituée de molécules en C6+ qui est envoyée dans l'unité d'hydrotraitement (30) en mélange avec la fraction lourde (5) issue de l'unité de séparation (21), l'effluent (31) de ladite unité d'hydrotraitement (30) constituant la coproduction d'essence désulfurée à haut indice d'octane.

Dans certains cas, il est possible d'extraire de la ou des unités de distillation (50) une coupe (9b), majoritairement constituée de molécules en C6/C8, qui est envoyée dans une unité d'extraction des aromatiques (60), l'effluent (12) de la dite unité d'extraction des aromatiques (60) étant valorisé comme base pétrochimique. Une partie (13) de ce flux (12) peut éventuellement être mélangé avec le flux (31) pour constituer l'essence désulfurée à haut indice d'octane (14).

Les conditions opératoires de l'étape d'oligocraquage étant définies dans la revendication 1.

Dans une variante du procédé selon l'invention, l'ensemble des coupes de fond (9a) +(9b) constitué de molécules à plus de 6 atomes de carbone (noté C6+), est envoyé dans l'unité d'hydrotraitement (30) en mélange avec la fraction lourde (5) issue de l'unité de séparation (21), l'effluent (31) de la dite unité d'hydrotraitement (30) constituant la coproduction d'essence désulfurée à haut indice d'octane.

Le taux de recyclage de la coupe C5/C6 (flux 15+16) extraite de l'unité d'oligocraquage en une étape (40) est généralement compris entre 1 et 5, et préférentiellement compris entre 2 et 4.

Le catalyseur utilisé dans l'unité d'oligocraquage en une étape (40) comprend au moins une zéolithe présentant une sélectivité de forme, cette zéolithe ayant un rapport atomique Si /Al compris entre 50 et 500, et préférentiellement compris entre 75 et 150.

Le catalyseur utilisé dans l'unité d'oligocraquage en une étape (40) peut être une zéolithe à sélectivité de forme appartenant à un premier groupe constitué par l'un des types structuraux suivants: MEL, MFI, NES, EUO, FER, CHA, MFS, MWW, ou être constitué d'un mélange quelconque des éléments de ce premier groupe.

Le catalyseur utilisé dans l'unité d'oligocraquage en une étape (40) peut également être une zéolithe à sélectivité de forme appartenant à un second groupe constitué par les zéolithes suivantes: NU-85, NU-86, NU-88 et IM-5 ou être constitué d'un mélange quelconque des éléments de ce second groupe.

L'unité d'oligocraquage en une étape (40) travaille généralement aux conditions opératoires suivantes:

> température comprise entre 450°C et 580°C, pression comprise entre 0,1 MPa et 0,5 MPa, et vitesse spatiale comprise entre 1 h⁻¹ et 10 h⁻¹ par rapport à la charge fraîche entrant dans l'unité ( flux (6)).

Le procédé de production de propylène et de coproduction d'une essence désulfurée à haut indice d'octane selon l'invention comporte de façon optionnelle une unité d'extraction des aromatiques (60) traitant le flux majoritairement aromatique (9b) extrait de l'unité de séparation par distillation (50). Une partie (13) de l'effluent de la dite unité d'extraction des aromatiques (60) peut être mélangé à l'effluent (31) de l'unité d'hydrotraitement (30) pour former la coproduction d'essence désulfurée à haut indice d'octane (14).

**Description détaillée de l'invention**

La charge du procédé selon l'invention est constituée de deux composants, une coupe C4/C5 oléfinique ex vapocraquage (A) repérée par le flux (1), et une coupe essence ex craquage catalytique (B), repérée par le flux (2) et notée essence ex FCC dans la suite de la description.

L'essence ex FCC (B) est envoyée dans une unité d'hydrogénation sélective (20) travaillant dans des conditions opératoires classiques pour ce type d'unité et sur un catalyseur qui est préférentiellement à base d'un élément du groupe VIII déposé sur une alumine γ. L'objectif de cette unité d'hydrogénation sélective (20) est d'éliminer les doubles liaisons caractérisants les dioléfines.

La surface acide externe du catalyseur ne doit pas être trop importante pour limiter les réactions de polymérisation à la surface du catalyseur. Lorsque c'est le nickel qui est utilisé comme métal, sa teneur est comprise entre 5% et 25 % poids, préférentiellement entre 7% et 20 % poids. Le catalyseur est sulfuré afin de passiver les atomes de nickel de surface.

Les conditions opératoires de l'unité d'hydrogénation sélective sont choisies pour que l'effluent reste à l'état liquide, soit de 120°C à 200 °C, sous des pressions allant de 0,5 MPa à 40 MPa. La quantité de catalyseur utilisé pour effectuer la réaction d'hydrogénation sélective se situe typiquement entre 2 m³ et 8 m³ de catalyseur par m³ de charge fraîche traitée.

L'hydrogène est introduit à hauteur de 5 % à 30 % molaire au dessus de la quantité stoechiométrique, et préférentiellement de 10 % à 20 % au dessus de la quantité stoechiométrique.

L'effluent (3) de l'unité d'hydrogénation sélective (20) est envoyé dans une unité de distillation (21) qui permet de séparer l'effluent en une fraction légère (4) et une fraction lourde (5).

La fraction légère (4) constituée des composés carbonés à moins de 6 atomes de carbone est mélangée avec la coupe C4/C5 ex vapocraquage (1).

Le mélange résultant (6) est envoyé dans l'unité d'oligocraquage en une étape (40).

L'effluent (17) de l'unité d'oligocraquage en une étape est séparé dans une ou plusieurs colonnes à distiller (50) en au moins 5 flux.

- un flux de tête (10) constitué d'oléfines légères , majoritairement d'éthylène qui est valorisé comme base pétrochimique,
- un flux (7) qui est constitué essentiellement de propylène,
- un flux (8) majoritairement constitué de C4 saturés, qui est pour une partie (11) valorisé en tant que gaz de pétrole liquéfié (GPL), et dont une partie (16) est recyclée à l'entrée de l'unité d'oligocraquage en une étape (40).
- un flux intermédiaire (18) constituée d'une fraction ayant majoritairement de 5 à 6 atomes de carbone, qui est au moins en partie recyclée (flux 15) à l'entrée de l'unité d'oligocraquage en une étape (40), l'autre partie (flux 19) étant envoyée au pool essence,
- et un flux de fond (9a) constitué d'hydrocarbures à plus de 9 atomes de carbone qui est envoyé en mélange avec la fraction lourde (5) dans l'unité d'hydrotraitement (30), dont l'effluent (31) constitue la production d'essence désulfurée.
- un flux (9b) riche en aromatiques est envoyé de façon optionnelle dans une unité d'extraction des aromatiques (60) produisant un effluent (12), valorisable comme base pétrochimique. Une partie (13) de ce flux (12) peut être mélangé avec le flux (31) pour former l'essence désulfurée à haut indice d'octane (14),

Dans une variante du procédé selon l'invention l'ensemble des coupes de fond (9a) +(9b) constituée de molécules C6+ est envoyé dans l'unité d'hydrotraitement (30) en mélange avec la fraction lourde (5) issue de l'unité de séparation (21), l'effluent (31) de la dite unité d'hydrotraitement (30) constituant la coproduction d'essence désulfurée à haut indice d'octane.

L'unité d'extraction des aromatiques (60) est une unité classique, bien connue de l'homme du métier, par exemple une unité d'extraction utilisant comme solvant une solution contenant du diméthylsulfoxyde (DMSO) dont on trouvera une description dans l'ouvrage de Chauvel, Lefebvre et Castex ("Procédés de pétrochimie : caractéristiques techniques et économiques-Tome 1 - Edition Technip 1985).

La fraction lourde (5) issue de l'unité de séparation (21) est envoyée dans l'unité d'hydrotraitement (30) en mélange avec le flux de fond (9a) issue de la colonne à distiller (50). L'effluent (31) de l'unité d'hydrotraitement (30) peut être mélangé à une partie de l'effluent (13) de l'unité d'extraction des aromatiques (60) pour constituer l'essence désulfurée à haut indice d'octane (14) coproduite par le procédé.

Le catalyseur utilisé dans l'unité d'hydrotraitement (30) comprend au moins un élément du groupe VIII choisi dans le groupe constitué par le fer, le ruthénium, l'osmium, le cobalt, le rhodium, l'iridium, le nickel, le palladium ou le platine, et au moins un élément du groupe VIB choisi dans le groupe constitué par le chrome, le molybdène et le tungstène, chacun de ces éléments se trouvant au moins en partie sous forme de sulfure.

Le catalyseur utilisé dans l'unité d'hydrotraitement (30) comprend entre 0,5% et 15% poids de métal du groupe VIII, ce pourcentage étant exprimé sous forme d'oxyde.

La teneur en poids de métal du groupe VIB est généralement comprise entre 1,5% et 60% poids, et préférentiellement entre 2% et 50% poids.

L'élément du groupe VIII est préférentiellement du cobalt, et l'élément du groupe VIB est préférentiellement le molybdène ou le tungstène.

Le support du catalyseur utilisé dans l'unité d'hydrotraitement est habituellement un solide poreux, tel que par exemple de la magnésie, de la silice, de l'oxyde de titane ou de l'alumine, seuls ou en mélange.

La température opératoire de l'unité d'hydrotraitement (30) est généralement comprise entre 220°C et 340°C, sous une pression comprise entre 1 MPa et 5 MPa (1MPa = 10 bars).

La vitesse spatiale horaire est comprise entre environ $1h^{-1}$ et $20\ h^{-1}$. La vitesse spatiale est définie comme le volume de charge par volume de catalyseur et par heure.

Le rapport du débit d'hydrogène sur le débit de charge est compris entre 100 litres/litre et 600 litres/litre, exprimé en normaux litres d'hydrogène par litre d'essence.

Typiquement, le catalyseur utilisé dans l'unité d'oligocraquage en une étape (40) comprend au moins une zéolithe présentant une sélectivité de forme, cette zéolithe ayant un rapport atomique Si /Al compris entre 50 et 500, et préférentiellement compris entre 75 et 150.

L'expression "sélectivité de forme" signifie que les zéolithes utilisées dans la présente invention présentent une sélectivité induite par leur structure poreuse de taille moléculaire.

La sélectivité de forme repose essentiellement sur l'impossibilité pour certaines molécules de réactif de pénétrer dans les pores et/ou pour certains produits formés de sortir desdits pores.

En outre, la zéolithe présentant une sélectivité de forme, peut appartenir à un premier groupe constitué par l'un des types structuraux suivants: MEL, MFI, NES, EUO, FER, CHA, MFS, MWW. Elle peut être également constituée d'un mélange quelconque des différents éléments de ce premier groupe.

La zéolithe à sélectivité de forme peut également appartenir à un second groupe constitué par les zéolithes suivantes: NU-85, NU-86, NU-88 et IM-5. La zéolithe peut être également constituée d'un mélange quelconque des éléments de ce second groupe.

L'un des avantage de ces zéolithes présentant une sélectivité de forme est qu'il conduit à une meilleure sélectivité propylène / isobutène, c'est à dire à un rapport propylène / isobutène plus élevé dans les effluents de la dite unité d'oligocraquage.

La ou les zéolithes utilisées dans l'unité d'oligocraquage en une étape (40) peuvent être dispersées dans une matrice à base de silice, zircone, alumine ou de silice alumine, la proportion de zéolithe(s) étant généralement comprise entre 15% et 90 % poids, de préférence entre 30 % et 80 % poids.

Des rapports atomiques Si/Al compris dans la fourchette préférée dans le cadre de l'invention peuvent être obtenus au moment de la fabrication de la zéolithe, ou par désalumination ultérieure.

On peut notamment utiliser l'une des zéolithes commerciales ZSM-5 suivantes:

- la CBV 28014 (rapport Si/Al :140), et la CBV 1502 (rapport atomique Si/Al :75) de la société Zeolyst International, Valley Forge PA., 19482 USA,
- la ZSM-5 Pentasil de rapport atomique Si/Al 125 de Süd-Chemie (Munich, Allemagne).

Le catalyseur utilisé dans l'unité d'oligocraquage en une étape (40) est généralement mis en oeuvre en lit mobile, préférentiellement sous forme de billes de diamètre généralement compris entre 1 mm et 3 mm.

Le catalyseur de l'unité d'oligocraquage en une étape (40) peut également être mis en oeuvre à l'état de lit fixe, auquel cas le ou les réacteurs utilisés travaillent alternativement en réaction puis en régénération.

La phase de régénération comprend typiquement une phase de combustion des dépôts carbonés formés sur le catalyseur, par exemple à l'aide d'un mélange air/azote ou d'air appauvri en oxygène (par exemple par recirculation de fumées), ou simplement d'air.

La régénération peut éventuellement comprendre d'autres phases de traitement et de régénération du catalyseur qui ne seront pas développées ici, ne s'agissant pas d'un aspect caractéristique de l'invention.

On opère habituellement l'unité d'oligocraquage en une étape (40) à une température d'environ 450°C à environ 580°C, avec une vitesse spatiale (ppH) comprise généralement entre 1 h$^{-1}$ et 10 h$^{-1}$ par rapport à la charge fraîche (flux 6) entrant dans l'unité. La vitesse spatiale est définie comme la masse de charge rapportée à la masse de catalyseur et par heure.

La pression opératoire est généralement comprise entre 0,1 MPa et 0,5 MPa.

Les conditions de régénération du catalyseur d'oligocraquage utilisent généralement une température comprise entre 400°C et 650°C, la pression étant le plus souvent voisine de la pression utilisée pour la réaction d'oligocraquage.

Le débit de recyclage de la coupe C4/C6 (somme des flux 15 et 16) issue de l'unité d'oligocraquage en une étape (40) rapporté au débit de charge entrante (6) dans ladite unité à la dite unité peut varier dans un rapport de 1 à 5, et préférentiellement de 2 à 4.

Généralement, le rendement par passe en propylène (flux 7) rapporté à la quantité d'oléfines contenues dans la charge fraîche (flux 6) du procédé est supérieur à 19 %, et de préférence supérieur à 22% poids.

## Exemple selon l'invention

L'exemple ci dessous donne un bilan matière simplifié du schéma de procédé selon l'invention tel qu'illustré par la figure 1.

La coupe C4/C5 ex vapocraquage (A) a la composition suivante:

| Composants | Débit kg/h |
|---|---|
| nC4= | 9 672 |
| iC4= | 4 413 |
| Diènes | 22 |
| Paraffines C4 | 5 893 |
| n+i C5= | 6 500 |
| CycloC5 | 2 500 |
| Paraffines C5 | 1 000 |

La coupe essence ex FCC (B) a la composition suivante:

| Flux | B |
|---|---|
| Débit kg/h | 29227 |
| Densité (d15) | 0,735 |
| Soufre (ppm poids) | 380 |
| RHS (ppm poids) | --- |
| MAV* | 3,9 |
| Nombre de brome | 90 |
| Teneur en oléfines (%vol) | 45 |
| RON | 93,2 |
| MON | 80,5 |
| ASTM D86 point 5% | 52°C |
| ASTM D86 point 50% | 92°C |

(suite)

| Flux | B |
|------|---|
| ASTM D86 point 95% | 154°C |

| * MAV (Maleic Anhydride Value) dont la traduction en français est Indice d'Anhydride Maleïque) s'exprime en milligramme (mg) d'anhydride maleïque réagissant avec les dioléfines (diènes conjugués) contenues dans un gramme d'essence selon la méthode normalisée IFP N° 9407. |
|---|

L'unité d'oligocraquage en une étape (40) fonctionne avec un catalyseur zéolithique de type ZSM5 de rapport atomique Si/Al de 140, ladite zéolithe étant dispersée dans une matrice $Al_2O_3$. La proportion de zéolithe s'élève à 50% poids. L'unité d'oligocraquage en une étape (40) fonctionne aux conditions opératoires suivantes:

$$température = 510°C$$

$$pression = 0,02\ MPa$$

$$PPH = \frac{\text{Débit charge fraiche}}{\text{masse de catalyseur}} = 6,7 h^{-1}$$

Le débit de recyclage de la coupe C4/C6, soit la somme des flux (15 et 16) issue de l'unité d'oligocraquage en une étape (40), rapporté au débit de charge entrante (6), est de 2,8.

L'unité d'hydrotraitement (30) fonctionne avec un catalyseur NiMo sulfuré sur alumine de type HR 806 commercialisé par la société AXENS, aux conditions opératoires suivantes:

température: 290°C
pression: 2 MPa

$$VVH = 4\ h^{-1}$$

$$H2/HC = 360\ litres/litre$$

L'unité d'hydrogénation sélective (20) fonctionne avec un catalyseur HR 845 (commercialisé sous ce nom par la société AXENS) aux conditions opératoires suivantes:

température: 143 °C
pression: 3,2 MPa
VVH : 6 $h^{-1}$
H2/HC: 5 litres/litre

L'effluent de l'unité d'hydrogénation sélective (20) est envoyé dans une unité de distillation (21) qui sépare le flux (3) en une fraction légère (4) et une fraction lourde (5) dont les caractéristiques sont données dans le tableau 1 ci dessous:

Tableau 1 : Performance de l'unité d'hydrogénation sélective

| Flux | 4 | 5 |
|---|---|---|
| Débit kg/h | 10 377 | 18850 |
| Rendement (%) /flux B | 35,5 | 64,5 |
| Densité (d15) | 0,664 | 0,774 |
| Soufre (ppm poids) | 48 | 641 |
| RHS (ppm poids) | 0 | |
| MAV | 0 | 3 |
| Nombre de brome | 115 | 91 |
| RON | 96,3 | 91,5 |
| MON | 81,9 | |
| ASTM D86 point 5% | 29 | 100 |
| ASTM D86 point 50% | 44 | 119 |
| ASTM D86 point 95% | 64 | 161 |

L'unité d'extraction des aromatiques (60) est une unité utilisant comme solvant une solution contenant du diméthylsulfoxyde (DMSO) dont on trouvera une description dans l'ouvrage de Chauvel, Lefebvre et Castex "Procédés de pétrochimie/ caractéristiques techniques et économiques" Tome 1 - Edition Technip 1985.

Les débits des différents flux selon la figure 1 sont donnés dans le tableau 2 ci dessous.

Le tableau 3 récapitule le bilan matière selon les différents flux de la figure 1, rapporté à l'ensemble des charges entrantes (A+B), soit les flux (1+2).

Le rendement en propylène (flux 7) rapporté à l'ensemble des charges entrantes (A+B), soit les flux (1+2) est de 22,1%.

Le rendement en essence désulfurée (flux 14) à haut MON rapporté à l'ensemble des charges entrantes (A+B) (flux 1+2) est de 35,4%.

Le MON de l'essence produite (flux 14) est de 80,5. Le RON est de 92,7.

La teneur en soufre de l'essence produite (flux 14) est inférieure à 50 ppm.

Tableau 2: débits massiques (kg/h) des flux selon la figure 1

| Flux | 10 | 7 | 11 | 19 | 12 | 14 |
|---|---|---|---|---|---|---|
| C1+C2 | 212 | | | | | |
| C2= | 2888 | | | | | |
| C3 | | 957 | | | | |
| C3= | | 13099 | | | | |
| nC4= | | | 1312 | | | |
| iC4= | | | 842 | | | |
| Diènes C4 | | | 22 | | | |
| Paraffines C4 | | | 7845 | | | |
| n+i C5= | | | | 606 | | |
| Cyclo C5 | | | | 15 | | |
| paraffines C5 | | | | 5126 | | |
| C6= | | | | 107 | | |
| C6 | | | | 934 | | |
| Essence désulfurée : à haut MON | | | | | | 20953 |
| Bases pétro (BTX) | | | | | 4234 | |

**Tableau 3 : rendements globaux rapportés à la somme des charges A+B**

| Flux | | Rdt/ (Flux 1+2) |
|------|------|------|
| 10 | C1+C2<br>C2= | 5,2<br>4,9 |
| 7 | C3= | 22,1 |
| 11 | C4 | 16,9 |
| 19 | C5C6 | 11,5 |
| 12 | BTX | 7,1 |
| 14 | Essence à haut MON | 35,4 |

Les caractéristiques de l'essence désulfurée à haut indice d'octane (flux 14) sont données dans le tableau 4 ci dessous:

**Tableau 4: caractéristiques de l'essence désulfurée à haut indice d'octane**

| Flux | 14 |
|------|------|
| Débit (kg/h) | 20953 |
| Densité (d15) | 0,733 |
| Soufre (ppm poids) | <50 |
| RHS (ppm poids) | --- |
| MAV | <0.5 |
| Nombre de brome (g*100g) | 78 |
| Teneur en oléfines (%vol) | 40 |
| RON | 92,7 |
| MON | 80,5 |

**Revendications**

1. Procédé de production de propylène et de coproduction d'une essence désulfurée à haut indice d'octane à partir d'une charge constituée d'une part d'une coupe C4/C5 issue d'une unité de vapocraquage (A), et d'autre part d'une essence de FCC (B), le dit procédé comportant les étapes suivantes:

   - une étape d'hydrogénation sélective (20) de l'essence de FCC (B) (flux 2), produisant un effluent (3) envoyé dans une unité de séparation (21) qui produit deux fractions, une fraction légère (4) qui est envoyée dans une unité d'oligocraquage en une étape (40), et une fraction lourde majoritairement en C6+ (5), qui est envoyée dans une unité d'hydrotraitement (30),
   - une étape d'oligocraquage en une étape (40) qui traite en mélange la coupe C4/C5 issue de l'unité de vapocraquage (A) (flux 1), et la fraction légère (4) issue de l'unité de séparation (21), ladite étape d'oligocraquage travaillant aux conditions opératoires suivantes:

   température comprise entre 450°C et 580°C, pression comprise entre 0,1 MPa et 0,5 MPa, et vitesse spatiale comprise entre 1 h$^{-1}$ et 10 h$^{-1}$ par rapport à la charge fraîche entrant dans l'unité d'oligocraquage (flux 6),
   l'effluent (17) de l'unité d'oligocraquage (40) étant séparé dans une ou plusieurs colonnes à distiller (50) en au moins 5 coupes:

   1) une coupe de tête (10) constituée majoritairement d'éthylène

2) une coupe de tête (7) qui constitue la production en propylène du procédé,

3) une coupe intermédiaire (8) constituée majoritairement de molécules en C4, dont une partie (16) est recyclée à l'entrée de l'unité d'oligocraquage en une étape (40), et dont la partie non recyclée (11) est valorisée en gaz de pétrole liquéfié,

4) une coupe intermédiaire (18) constituée majoritairement de molécules en C5 et C6, dont une partie (15) est recyclée à l'entrée de l'unité d'oligocraquage en une étape (40), et dont la partie non recyclée (19) constitue une essence dirigée vers le pool essence,

5) une coupe de fond (9a) constituée de molécules en C9+ qui est envoyée dans l'unité d'hydrotraitement (30) en mélange avec la fraction lourde (5) issue de l'unité de séparation (21), l'effluent (31) de ladite unité d'hydrotraitement (30) constituant la coproduction d'essence désulfurée à haut indice d'octane.

2. Procédé de production de propylène et de coproduction d'une essence désulfurée à haut indice d'octane selon la revendication 1, dans lequel on extrait de la ou des colonnes à distiller (50) une coupe (9b), majoritairement constituée de molécules en C6/C8, qui est envoyée dans une unité d'extraction des aromatiques (60), l'effluent de la dite unité d'extraction des aromatiques (60) étant pour une partie (12) valorisé comme base pétrochimique, et pour une autre partie (13) envoyé en mélange avec l'effluent (31) de l'unité d'hydrotraitement (30) pour constituer l'essence désulfurée à haut indice d'octane (14).

3. Procédé de production de propylène et de coproduction d'une essence désulfurée à haut indice d'octane selon l'une quelconque des revendications 1 à 2, dans lequel le taux de recyclage de la coupe C5/C6 (15 +16) extraite de l'unité d'oligocraquage en une étape (40) est compris entre 1 et 5, et préférentiellement compris entre 2 et 5.

4. Procédé de production de propylène et de coproduction d'une essence désulfurée à haut indice d'octane selon l'une quelconque des revendications 1 à 3, dans lequel le catalyseur utilisé dans l'unité d'oligocraquage en une étape (40) comprend au moins une zéolithe présentant une sélectivité de forme, cette zéolithe ayant un rapport atomique Si /Al compris entre 50 et 500, et préférentiellement compris entre 75 et 150.

5. Procédé de production de propylène et de coproduction d'une essence désulfurée à haut indice d'octane selon l'une quelconque des revendications 1 à 4, dans lequel le catalyseur utilisé dans l'unité d'oligocraquage en une étape (40) est une zéolithe à sélectivité de forme appartenant à un premier groupe constitué par l'un des types structuraux suivants: MEL, MFI, NES, EUO, FER, CHA, MFS, MWW, ou est constitué d'un mélange quelconque des éléments de ce premier groupe.

6. Procédé de production de propylène et de coproduction d'une essence désulfurée à haut indice d'octane selon l'une quelconque des revendications 1 à 5, dans lequel le catalyseur utilisé dans l'unité d'oligocraquage en une étape (40) est une zéolithe à sélectivité de forme appartenant à un second groupe constitué par les zéolithes suivantes: NU-85, NU-86, NU-88 et IM-5 ou est constitué d'un mélange quelconque des éléments de ce second groupe.

7. Procédé de production de propylène et de coproduction d'une essence désulfurée à haut indice d'octane selon l'une quelconque des revendications 1 à 6 dans lequel le catalyseur utilisé dans l'unité d'oligocraquage en une étape (40) est mis en oeuvre en lit mobile, préférentiellement sous forme de billes de diamètre compris entre 1 mm et 3 mm.

**Claims**

1. A process for producing propylene and co-producing a desulphurized gasoline with a high octane number from a feed constituted by a C4/C5 cut from a steam cracking unit (A) and a FCC gasoline (B), said process comprising the following steps:

• a step for selective hydrogenation (20) of the FCC gasoline (B) (stream 2), producing an effluent (3) sent to a separation unit (21) which produces two fractions, a light fraction (4) which is sent to an one-step oligocracking unit (40) and a mainly C6+ heavy fraction (5) which is sent to a hydrotreatment unit (30);

• a one step oligocracking step (40) which treats a mixture of C4/C5 cut from the steam cracking unit (A) (stream 1), and the light fraction (4) from the separation unit (21), the so called one step oligocracking step (40) having the following operating conditions, a temperature in the range 450°C to 580°C, a pressure in the range 0.1 MPa to 0.5 MPa, and an hourly space velocity in the range 1 $h^{-1}$ to 10 $h^{-1}$ with respect to fresh feed entering the oligocracking unit (stream 6),

the effluent (17) from the oligocracking unit (40) being separated in one or more distillation columns (50) into at least 5 cuts:

> 1) an overhead cut (10) mainly constituted by ethylene,
> 2) an overhead cut (7) which constitutes the propylene production of the process,
> 3) an intermediate cut (8) mainly constituted by C4 molecules, a portion (16) of which is recycled to the inlet to the one-step oligocracking unit (40), and a non-recycled portion (11) of which is upgraded to liquefied petroleum gas,
> 4) an intermediate cut (18) mainly constituted by C5 and C6 molecules, a portion (15) of which is recycled to the inlet to the one step oligocracking unit (40), the non-recycled portion (19) constituting a gasoline directed to the gasoline pool,
> 5) a bottom cut (9a) constituted by C9+ molecules which is sent to a hydrotreatment unit (30) mixed with the heavy fraction (5) from the separation unit (21), the effluent (31) from said hydrotreatment unit (30) constituting the co-production of desulphurized gasoline with a high octane number.

2. A process for producing propylene and co-producing a high octane number desulphurized gasoline according to claim 1, in which a cut (9b) mainly constituted by C6/C8 molecules is extracted from the distillation column or columns (50) which is sent to an aromatics extraction unit (60), a portion (12) of the effluent from said aromatics extraction unit (60) being upgraded as a petrochemicals base, and another portion (13) being sent as a mixture with the effluent (31) from the hydrotreatment unit (30) to constitute the high octane number desulphurized gasoline (14).

3. A process for producing propylene and co-producing a high octane number desulphurized gasoline according to claim 1 or claim 2, in which the recycle ratio of the C5/C6 cut (15+16) extracted from the one step oligocracking unit (40) is in the range 1 to 5, preferably in the range 2 to 5.

4. A process for producing propylene and co-producing a high octane number desulphurized gasoline according to any one of claims 1 to 3, in which the catalyst used in the one step oligocracking unit (40) comprises at least one zeolite having form selectivity, said zeolite having a Si/Al atomic ratio in the range 50 to 500, preferably in the range 75 to 150.

5. A process for producing propylene and co-producing a high octane number desulphurized gasoline according to any one of claims 1 to 4, in which the catalyst used in the one step oligocracking unit (40) is a zeolite with form selectivity belonging to a first group constituted by one of the following structure types: MEL, MFI, NES, EUO, FER, CHA, MFS, MWW, or constituted by any mixture of the elements of said first group.

6. A process for producing propylene and co-producing a high octane number desulphurized gasoline according to any one of claims 1 to 5, in which the catalyst used in the one step oligocracking unit (40) is a zeolite with form selectivity belonging to a second group constituted by the following zeolites: NU-85, NU-86, NU-88 and IM-5 or is constituted by any mixture of the elements of said second group.

7. A process for producing propylene and co-producing a high octane number desulphurized gasoline according to any one of claims 1 to 5, in which the catalyst used in the one step oligocracking unit (40) is processed in a mobile bed unit preferably under the form of beads with a diameter ranging between 1 mm and 3 mm.

**Patentansprüche**

1. Verfahren zur Produktion von Propylen und zur Coproduktion eines entschwefelten Benzins mit hoher Oktanzahl aus einer Beschickung, die einerseits aus einem C4/C5-Schnitt, der aus einer Einheit zum Dampfcracken (A) stammt, und andererseits einem FCC-Benzin (B) besteht, wobei das Verfahren die folgenden Schritte umfasst:

> - einen Schritt der selektiven Hydrierung (20) des FCC-Benzins (B) (Strom 2), der einen Abfluss (3) produziert, der in eine Trennungseinheit (21) geschickt wird, die zwei Fraktionen produziert, eine leichte Fraktion (4), die in eine Einheit zum oligomeren Cracken in einem Schritt (40) geschickt wird, und in eine schwere Fraktion (5), überwiegend aus C6+, die in eine Einheit zur Hydrobehandlung (30) geschickt wird,
> - einen Schritt des oligomeren Crackens in einem Schritt (40), bei dem ein Gemisch aus dem C4/C5-Schnitt, der aus der Einheit zum Dampfcracken (A) (Strom 1) stammt, und der leichten Fraktion (4), die aus der Trennungseinheit (21) stammt, behandelt wird, wobei der Schritt des oligomeren Crackens unter den folgenden Betriebsbedingungen erfolgt:

Temperatur im Bereich zwischen 450 °C und 580 °C, Druck im Bereich zwischen 0,1 MPa und 0,5 MPa, und Raumgeschwindigkeit im Bereich zwischen 1 $h^{-1}$ und 10 $h^{-1}$, bezogen auf die frische Beschickung, die in die Einheit zum oligomeren Cracken (Strom 6) eintritt,

wobei der Abfluss (17) der Einheit zum oligomeren Cracken (40) in einer oder mehreren Destillationskolonnen (50) in mindestens 5 Schnitte getrennt wird:

1) einen Kopfschnitt (10), der überwiegend aus Ethylen besteht

2) einen Kopfschnitt (7), der die Propylenproduktion des Verfahrens darstellt,

3) einem Zwischenschnitt (8), der überwiegend aus C4-Molekülen besteht, wovon ein Teil (16) zum Eingang der Einheit zum oligomeren Cracken in einem Schritt (40) recycelt wird, und dessen nicht recycelter Teil (11) zu verflüssigtem Erdölgas veredelt wird,

4) einem Zwischenschnitt (18), der überwiegend aus C5- und C5-Molekülen besteht, wovon ein Teil (15) zum Eingang der Einheit zum oligomeren Cracken in einem Schritt (40) recycelt wird, und dessen nicht recycelter Teil (19) aus einem Benzin besteht, das zum Benzinpool geleitet wird,

5) einem Bodenschnitt (9a), der aus (C9+)-Molekülen besteht, der in eine Einheit zur Hydrobehandlung (30) geschickt wird, gemischt mit der schweren Fraktion (5), die aus der Trennungseinheit (21) stammt, wobei der Abfluss (31) dieser Einheit zur Hydrobehandlung (30) aus der Coproduktion von entschwefeltem Benzin mit hoher Oktanzahl besteht.

2. Verfahren zur Produktion von Propylen und zur Coproduktion eines entschwefelten Benzins mit hoher Oktanzahl nach Anspruch 1, wobei aus der oder den Destillationskolonnen (50) ein Schnitt (9b) extrahiert wird, der überwiegend aus C6/C8-Molekülen besteht, der in eine Einheit zur Extraktion von Aromaten (60) geschickt wird, wobei der Abfluss aus der Einheit zur Extraktion von Aromaten (60) zu einem Teil (12) als petrochemischer Grundstoff veredelt wird, und zum anderen Teil (13), gemischt mit dem Abfluss (31) der Einheit zur Hydrobehandlung (30) geschickt wird, um das entschwefelte Benzin mit hoher Oktanzahl (14) zu bilden.

3. Verfahren zur Produktion von Propylen und zur Coproduktion eines entschwefelten Benzins mit hoher Oktanzahl nach einem der Ansprüche 1 bis 2, wobei die Recyclingrate des C5/C6-Schnitts (15 + 16), der aus der Einheit zum oligomeren Cracken in einem Schritt (40) extrahiert wird, im Bereich zwischen 1 und 5, und vorzugsweise im Bereich zwischen 2 und 5 liegt.

4. Verfahren zur Produktion von Propylen und zur Coproduktion eines entschwefelten Benzins mit hoher Oktanzahl nach einem der Ansprüche 1 bis 3, wobei der Katalysator, der in der Einheit zum oligomeren Cracken in einem Schritt (40) verwendet wird, mindestens einen Zeolithen umfasst, der eine Formselektivität aufweist, wobei dieser Zeolith ein Si/Al-Atomverhältnis im Bereich zwischen 50 und 500, und vorzugsweise im Bereich zwischen 75 und 150 aufweist.

5. Verfahren zur Produktion von Propylen und zur Coproduktion eines entschwefelten Benzins mit hoher Oktanzahl nach einem der Ansprüche 1 bis 4, wobei der Katalysator, der in der Einheit zum oligomeren Cracken in einem Schritt (40) verwendet wird, ein Zeolith mit einer Formselektivität ist, der zu einer ersten Gruppe gehört, die aus einem der folgenden Strukturtypen: MEL, MFI, NES, EUO, FER, CHA, MFS, MWW besteht, oder der aus einem beliebigen Gemisch der Elemente dieser ersten Gruppe besteht.

6. Verfahren zur Produktion von Propylen und zur Coproduktion eines entschwefelten Benzins mit hoher Oktanzahl nach einem der Ansprüche 1 bis 5, wobei der Katalysator, der in der Einheit zum oligomeren Cracken in einem Schritt (40) verwendet wird, ein Zeolith mit einer Formselektivität ist, der zu einer zweiten Gruppe gehört, die aus den folgenden Zeolithen: NU-85, NU-86, NU-88 und IM-5 besteht, oder der aus einem beliebigen Gemisch der Elemente dieser zweiten Gruppe besteht.

7. Verfahren zur Produktion von Propylen und zur Coproduktion eines entschwefelten Benzins mit hoher Oktanzahl nach einem der Ansprüche 1 bis 6, wobei der in der Einheit zum oligomeren Cracken in einem Schritt (40) verwendete Katalysator in einem Bewegtbett vorzugsweise in Form von Kugeln mit einem Durchmesser im Bereich zwischen 1 mm und 3 mm eingesetzt wird.

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2608595
- WO 0104237 A
- WO 9929805 A
- EP 0921181 A
- EP 0921179 A
- EP 1195424 A1
- US 6049017 A
- US 20050222475 A

**Littérature non-brevet citée dans la description**

- Production of Propylène from Low Valued Olefins. Hydrocarbon Engineering'' (Ingénierie des hydrocarbures. Mai 1999
- **CHAUVEL ; LEFEBVRE ; CASTEX.** Procédés de pétrochimie : caractéristiques techniques et économiques. 1985
- **CHAUVEL ; LEFEBVRE ; CASTEX.** Procédés de pétrochimie/ caractéristiques techniques et économiques. 1985